# EUROPEAN PATENT SPECIFICATION

(11) **EP 2 775 924 B1**
(45) Date of publication and mention of the grant of the patent: **17.05.2017**
(21) Application number: 12806142.1
(22) Date of filing: 31.10.2012
(51) Int. Cl.: A61B 6/02, A61B 6/00

(54) **ENLARGED ANGULAR GATING WINDOW C-ARM IMAGE ACQUISITION**
C-BOGEN-BILDAUFNAHME MIT SYNCHRONISATIONSFENSTER MIT VERGRÖSSERTEM WINKELBEREICH
ACQUISITION D'IMAGES FAISANT APPEL À UN ARCEAU ET CARACTÉRISÉE PAR UNE FENÊTRE DE SYNCHRONISATION ANGULAIRE ÉLARGIE

(30) Priority: 11.11.2011 US 201161558468 P
(43) Date of publication of application: 17.09.2014
(73) Proprietor: Koninklijke Philips N.V., 5656 AE Eindhoven (NL); Philips Deutschland GmbH, 20099 Hamburg (DE)
(72) Inventor: GRASS, Michael, 5656 AE Eindhoven (NL); SCHÄFER, Dirk, 5656 AE Eindhoven (NL); BRENDEL, Bernhard Johannes, 5656 AE Eindhoven (NL); LORENZ, Cristian, 5656 AE Eindhoven (NL)
(74) Representative: Steffen, Thomas
(86) International application number: PCT/IB2012/056054
(87) International publication number: WO 2013/068888

(56) References cited:
- WO-A1-2009/104156
- WO-A1-2012/123843
- DE-A1-102010 011 663
- US-A1- 2010 329 416
- US-A1- 2011 075 814

## Description

### FIELD OF THE INVENTION

The present invention relates to a C-arm structure for X-ray imaging, an X-ray imaging system, a method for providing tomographic image data of an object, a computer readable program element and a computer readable medium.

### BACKGROUND OF THE INVENTION

In C-arm X-ray imaging, computer tomographic image data is provided along a trajectory. For example, in cardiac C-arm CT imaging, cardiac gating may be required during image reconstruction. This is used, for example, for 3D coronary angiography or 3D whole heart imaging. For example, document US 2010/0098315 A1 relates to C-arm image acquisition and describes iterative reconstruction of coronary arteries. However, due to the slow rotation time of a C-arm system compared to, for example, the temporal length of a cardiac cycle, cardiac gating with a finite gating window width may lead to an interrupted projection sequence available for reconstruction. In terms of reconstruction image quality, this means that by increasing the temporal length of the cardiac gating window, artefacts due to angular sub-sampling will decrease, while artefacts due to limited temporal resolution will increase, and vice versa for decreasing gating window length.Document US2011075814 represents the closest prior art to the subject-matter of the independent claims and discloses a rotational C-arm structure with an X-ray source and a detector, wherein the X-ray source comprises two focal spots spaced apart from each other in an offset direction aligned with the trajectory of the X-ray source during the rotation of the C-arm.

### SUMMARY OF THE INVENTION

Thus, there is a need to provide C-arm CT image acquisition with enlarged gating window.

The object of the present invention is solved by the subject-matter of the independent claims, wherein further embodiments are incorporated in the dependent claims.

It should be noted that the following described aspects of the invention apply also for the C-arm structure, the X-ray imaging system, the method for providing tomographic image data of an object, as well as to the computer program element and the computer readable medium.

According to a first aspect of the present invention, a C-arm structure for X-ray imaging is provided, comprising a C-arm, a movable C-arm support, an X-ray source, and an X-ray detector. The C-arm comprises a first end and a second end, wherein the X-ray source is mounted to the first end and the detector is mounted to the second end. The C-arm is mounted to the C-arm support such that the X-ray source and the detector are movable around an object of interest on respective trajectories being dual axis rotation trajectories based on a simultaneous propeller and roll movement of the C-arm. The X-ray source comprises at least a first focal spot and a second focal spot spaced apart from each other with a focal spot distance in an offset direction, which offset direction is aligned with the source trajectory.

The offset along the source trajectory allows the image acquisition from one position of the X-ray source for an enlarged field of view, namely not only by the acquisition of a first view from the first focal spot, but also with a second view from the second focal spot providing an additional angular view. With respect to the image acquisition during a gating window, for example, the tube may be moved with the same speed, e.g. in a stepwise manner, thus allowing image acquisition from a number of points. However, due to the additional, so-to-speak, extra view point, in form of the second focal spot, not only a larger number of views within the gating window can be provided, but also for a larger angular range, namely by the additional segment provided on behalf of the offset of the second focal spot.

According to an exemplary embodiment, the first and the second focal spot are positioned on the trajectory of the X-ray source.

According to a further exemplary embodiment, the offset distance and/or the offset direction are adaptable. For example, they are adapted during a scan along the trajectory.

According to a further exemplary embodiment, the X-ray source is provided as a stereo X-ray tube with two focal spots.

According to a further exemplary embodiment, the X-ray source is provided as a dual energy X-ray tube. For example, a first radiation with first spectra is supplied from the first focal spot, and a second radiation with second spectra is supplied from the second focal spot.

For example, the stereo tube may be a dual energy stereo X-ray tube.

Image acquisition may be provided for at least a part of the trajectory.

The offset distance may be provided in a fixed manner, or in an adaptable or variable manner. The offset direction is provided in an adaptable or variable manner. Thus, the orientation of the offset direction may be adaptable. For example, the offset direction may be aligned with the tangential of the source trajectory. The source and detector trajectories are each a dual axis rotation trajectory, wherein the trajectory is based on a simultaneous propeller and roll movement. For example, the trajectory is a so-called "XperSwing trajectory" by Philips.

The offset direction is adaptable relative to the source trajectory during the acquisition, for example the offset direction is rotatable. This can be provided, for example, by a rotatably mounted X-ray source and/or by electronic deflection thus adapting the positions of the focal spots.

According to a second aspect of the present invention, an X-ray imaging system is provided, comprising a C-arm structure according to one of the above mentioned C-arm structures, a moving arrangement for driving the C-arm structure and a processing unit. The processing unit is configured to control the moving arranged to actuate the movement of the X-ray source and the detector along the respective trajectories. The processing unit is configured to control the X-ray source and the generation of X-ray radiation from the first focal spot and the second focal spot. The processing unit is configured to control the detector and to receive raw image data from the detector.

The processing unit may be configured to control the X-ray source to radiate first X-ray radiation from the first focal spot towards the detector, and to radiate second X-ray radiation from the second focal spot towards the detector.

According to a third aspect of the present invention, a method for providing tomographic image data of an object is provided, comprising the following steps:
a) Moving an X-ray source on a first end of a C-arm structure along a source trajectory, wherein the X-ray source is provided with a first and a second focal spot that are displaced to each other with a focal spot distance in an offset direction, which offset direction is aligned with the source trajectory, and simultaneously moving a detector on a second end of the C-arm structure along a corresponding detector trajectory. wherein the source and detector trajectories are dual axis rotation trajectories based on a simultaneous propeller and roll movement of the C-arm;
b) Radiating an object with first X-ray radiation from the first focal spot towards the detector, or radiating the object with second X-ray radiation from the second focal spot towards the detector.
c) Detecting respective X-ray radiation from the first or the second X-ray radiation with the detector, and providing respective signals as raw image data.

The movement of step a) may be provided continuously.

The movement may be provided as a constant and uniform movement while steps b) and c) are performed. Steps b) and c) may be provided while step a) is being performed.

According to an exemplary embodiment, in step b), the first and the second X-ray radiation are radiated in an alternating manner.

According to a further exemplary embodiment, the first and second X-ray radiation is provided in a continuous alternating manner and a continuous sampling of raw image data is provided. A gating signal relating to a function of the object is provided during the continuous sampling in relation to the raw image data. From the continuously sampled raw image data, raw image data assigned to a predetermined gating signal phase is selected for a reconstruction of three-dimensional image data of the object. This is also referred to as "retrospective" evaluation for sampling.

According to an alternative exemplary embodiment, a gating signal relating to a function of the object is provided during the movement of the C-arm structure and the radiation of the object. The radiation and the detection are arranged only within predetermined gating signal phases. This is also referred to as "prospective" sampling or triggering.

The predetermined gating signal phases may be provided as gating windows. A number of gating windows may be provided along a predefined trajectory length.

For example, a trajectory of 180° is provided comprising at least five distributed positions for a gating window, for example twelve gating windows, i.e. twelve positions for a gating window may be provided.

The gating signal is, for example, an ECG signal of a patient under examination. The predetermined gating signal phase may be a predetermined section of a heart cycle, for example 20 % of a heart cycle.

According to an exemplary embodiment, X-ray radiation from one of the two focal spots is provided from a first number of positions across the whole trajectory. X-ray radiation by the other one of the two focal spots is provided from a second number of positions arranged in a number of gating windows across the whole trajectory.

According to a further exemplary embodiment, X-ray radiation from the first focal spot and the second focal spot is provided only from a number of positions arranged in a number of gating windows across the trajectory.

According to a further exemplary embodiment, when switching on the second focal spot only inside cardiac gating windows, a full rotational sequence can be acquired with one focal spot at for example 30 frames per second, while inside the gating window, the second focal spot is added leading to a total frame rate of 60 Hz.

According to a further exemplary embodiment, the offset distance is adapted during a rotational scan. For example, by adjusting the distance, different speed of the movement can be compensated, for example, during the acceleration at the beginning and the deceleration at the end of the trajectory.

The tube displacement between two succeeding radiation positions, in which the first and second radiations are provided each, can be smaller than the focal spot distance. For example, the moving of step a) is provided in relation to the radiation of step b) such that a pair of a first and a second effective source position is followed by a subsequent pair of a first and second effective source position arranged in a displaced manner such that one of the effective source positions of the subsequent pair is arranged between the former first and second effective source position and the other one of the effective source positions of the subsequent pair is arranged outside the displacement of the former first and second effective source position.

The tube displacement can also be equal to the focal spot distance, for example in dual energy mode to provide image information relating to both spectra from the same viewing position. In other words, the focal spots are arranged in an overlapping or matching manner concerning succeeding (pair-) acquisitions.

The tube displacement can further be larger than the focal spot distance.

According to a further example, X-ray radiation is provided with dual energy X-ray radiation. For example, the first radiation is supplied with first spectra, and the second radiation is supplied with second spectra. The spectra analysis can be performed in the projection space or in the image space.

According to an aspect of the present invention, an enlarged gating window is provided by providing a second focal spot displaced in the direction of the trajectory. Thus, while applying the same speed of movement, additional image data of the heart, for example, may be provided, which improves the projection sequence available for reconstruction, thus reducing the angular subsampling, i.e. reducing the interrupted effect. Further, also the temporal width of the gating window may be kept unchanged, although an additional angular range of the gated projection acquisition is achieved by the second focal spot according to the present invention.

According to a further aspect, for example the focal spot distance may be up to 4 cm. Assuming a projection acquisition with for example 30 frames per second, and a projection acquisition using the focal spot positions in an alternating manner, the subsequent projections are acquired at an angular distance of about 2.74°. For a coronary angiography reconstruction in an acquisition with a single focal spot, a cardiac gating window is typically 20 % of the cardiac cycle length (the RR-interval), which translates into a temporal length of 200 msec for a typical heart rate of 60 beats per minute. A 180° rotational acquisition may be acquired in 5 to 7 sec and thus the angular range covered inside the gating window is about 5.14° for each gating window (when applying the acquisition in 7 sec). Thus, using a second focal spot, according to the present invention, and alternating projection acquisition from the two focal spots, the potential to increase the angular range of the gating window is approximately 50% at equal temporal resolution of the acquired data. Thus, it is possible to deliver significantly improved image quality.

These and other aspects of the invention will become apparent from and will be elucidated with reference to the embodiments described hereinafter.

### BRIEF DESCRIPTION OF THE DRAWINGS

Exemplary embodiments of the invention will be described in the following with reference to the following drawings.
Fig. 1 schematically shows an X-ray imaging system according to an exemplary embodiment of the present invention.
Fig. 2 shows a C-arm structure for X-ray imaging according to an exemplary embodiment of the present invention.
Fig. 3 schematically shows image acquisition along a trajectory with a C-arm structure according to an exemplary embodiment of the present invention.
Fig. 4 shows a further example of an X-ray image acquisition along a trajectory with a C-arm structure according to the present invention.
Figs. 5A to 5C schematically describe a further example of a C-arm structure according to the present invention, and possible trajectories.
Fig. 6 shows basic steps of a method for providing tomographic image data of an object according to an exemplary embodiment.
Fig. 7 to 10 show further examples of methods according to the present invention.

### DETAILED DESCRIPTION OF EMBODIMENTS

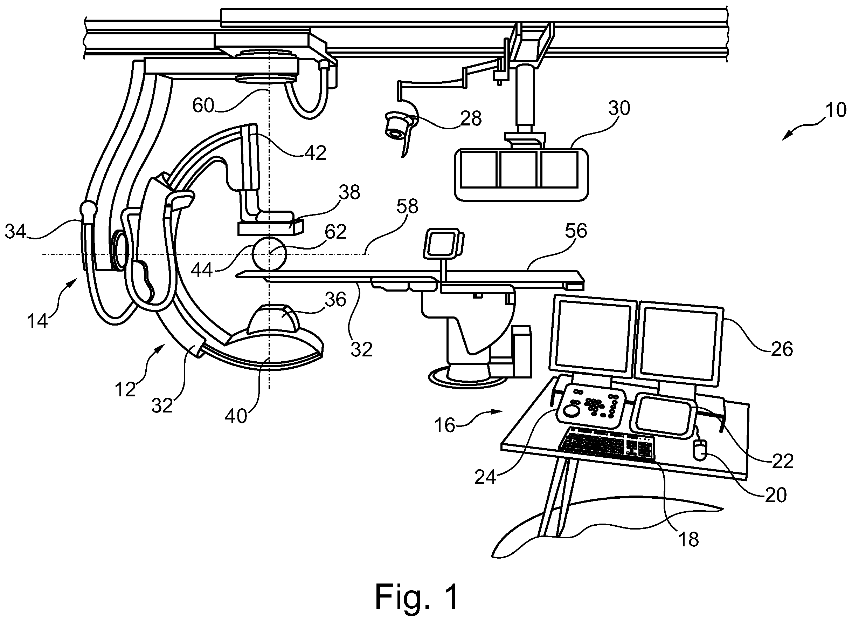

Fig. 1 shows an X-ray imaging system 10 with a C-arm structure 12. Further, a moving arrangement 14 for driving the C-arm structure is provided. Still further, a processing unit 16 is shown.

The processing unit 16 is provided with interfaces, such as a keyboard 18, a mouse 20, a graphic tablet 22, as well as further control input unit 24, and a monitor unit 26. However, it must be noted that according to the present invention, these peripheral interfaces are not necessarily part of the X-ray imaging system 10. Similar applies to lighting equipment 28 and a further display unit 30.

The C-arm structure 12 for X-ray imaging comprises a C-arm 32, a movable C-arm support 34, an X-ray source 36, and an X-ray detector 38.

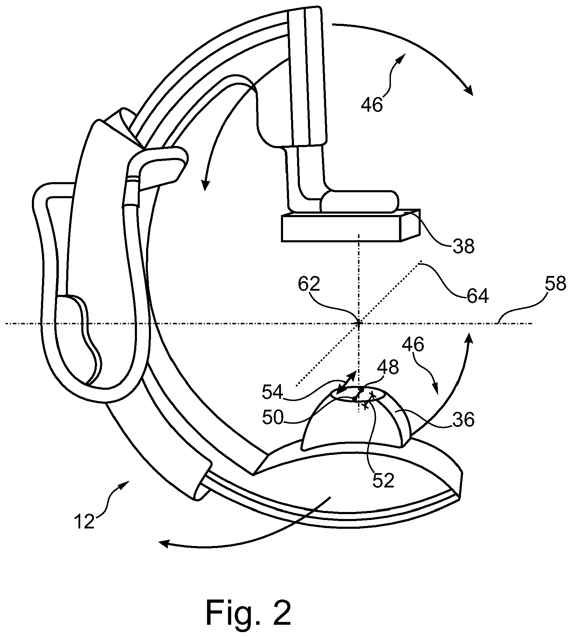

The C-arm 32 comprises a first end 40 and a second end 42. The X-ray source 36 is mounted to the first end 40 and the detector 38 is mounted to the second end 42. The C-arm 32 is mounted to the C-arm support 34 such that the X-ray source 36 and the detector 38 are movable around an object 44 of interest on respective trajectories 46, which are further described in relation with Fig. 2.

The X-ray source 36 comprises at least a first focal spot 48 and a second focal spot 50 spaced apart from each other with a focal spot distance 52 in an offset direction, indicated with double arrow 54, which offset direction 54 is aligned with the trajectory 46. This is shown in Fig. 2.

With reference to Fig. 1, it is further noted that the object 44 is provided on an object support, for example a patient table 56. The patient table 56 can be adjusted in a longitudinal direction, in height, and also in a rotational manner in the horizontal plane, which is not further shown, to adjust the position of the object to be arranged in a so-called ISO-centre, indicated by a first horizontal rotation axis 58 and a second vertical rotation axis 60, a cross-section 62 thereof being the ISO-centre.

It is noted further, that the present invention also relates to other devices in which a detector and an X-ray source are arranged opposite to each other such that an object to be examined can be arranged between the source and the detector, and wherein the detector and the X-ray source can be moved together in a common movement. For example, such X-ray detector and X-ray source arrangement and the respective movement can also be provided by a so-called O-arm structure, in which instead of a "C" an "O" is provided. As a further example, also two independent robotic arms can be provided for the source and the detector, which are configured to provide the respective movement of a C-arm structure.

The processing unit 16 is configured to control the moving arrangement 14 to actuate the movement of the X-ray source 36 and the detector 38 along the respective trajectories 46. The processing unit 16 is further configured to control the X-ray source and the generation of X-ray radiation from the first focal spot 48 and the second focal spot 50. The processing unit is still further configured to control the detector 38 and to receive raw image data from the detector 38.

The processing unit 16 is further configured to control the X-ray source 36 to radiate first X-ray radiation from the first focal spot 48 towards the detector 38, or to radiate second X-ray radiation from the second focal spot 50 towards the detector 38.

In Fig. 2, the C-arm structure 12 is shown in relation to the above mentioned trajectories 46. It must be noted that the trajectories 46 are symbolically shown as a rotational movement around the ISO-centre 62 around the horizontal axis 58.

However, also other rotational movements are provided according to the present invention, such as rotations around a horizontal axis 64 arranged transverse to the first horizontal axis 58.

Image acquisition may be provided for at least a part of the trajectory 46.

The first and the second focal spots are displaced in relation to each other in the direction of the trajectory 46. In other words, the offset direction 54 and the trajectory 46 are arranged in the same direction, according to an example.

The trajectory 46 may be a circular arc arranged in a trajectory plane. The offset direction is adaptable relative to the trajectory during the acquisition. The offset direction may further be rotatable, e. g. by a rotatably mounted X-ray source (not further shown), and/or by electronic deflection to adapt the position of the two focal spots, respectively. A simultaneous propeller and roll movement of the C-arm 32 is provided.

The offset distance 52 may be provided in a fixed manner. The offset distance 52 may also be provided in an adaptable or variable manner. The offset direction is provided in an adaptable or variable manner.

In the case the trajectory is not arranged in an even trajectory plane, the offset direction 54 is aligned with the tangential of the trajectory.

According to a further example, the first and the second focal spot 48, 50 are positioned on the trajectory 46 of the X-ray source 36.

According to an exemplary embodiment of the present invention, the X-ray source 36 in Fig. 2 may be a stereo X-ray tube with two focal spots. The stereo tube may be an X-ray tube with a single cathode providing a single electron beam, which electron beam is deflected to the two different focal spots 48, 50. The stereo tube may also be an X-ray tube with two cathodes adapted to alternately providing two different electron beams, which electron beams are deflected to a respective focal spot of the two focal spots 48, 50.

According to an exemplary embodiment, the X-ray source 36 of Fig. 2 may be a dual energy X-ray source, for example a dual energy X-ray tube. In case of a stereo tube, the tube may be a dual energy stereo tube.

According to an aspect of the present invention, the provision of two focal spots arranged in an offset in the direction of the trajectory is provided for cardiac C-arm CT imaging. This image acquisition procedure is used, for example, for 3D coronary angiography or for 3D whole heart imaging. Thus, the present invention is applicable for a number of clinical research applications in interventional cardiology, for example.

To achieve sharp images, cardiac gating is required during image reconstruction. Typically, the ECG is used as a gating signal.

It is noted that such an ECG signal providing arrangement may be part of the X-ray imaging system 10 shown in Fig. 1 (although) not further shown.

Due to the slow rotation time of the C-arm system compared to the temporal length of a cardiac cycle, cardiac gating with a finite gating window width is provided. According to the present invention, the gating window is enlarged, without increasing the temporal width, and also without an increase in the speed of the movement.

Higher speed of the C-arm system would have the disadvantage that larger actuating forces and also larger braking forces are required, and also implies the danger that staff, for example a surgeon, in the vicinity of the patient may be hit by a very fast moving C-arm structure.

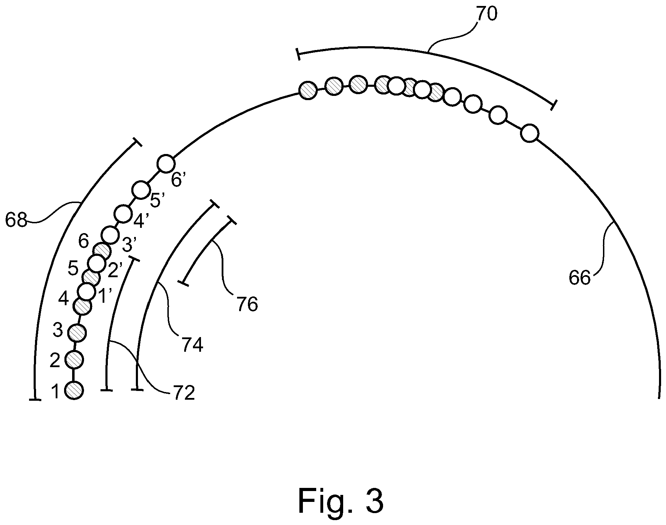

In Fig. 3, an example of a trajectory 66 is shown as a semicircle trajectory. Along the trajectory 66, a first gating window 68 and a second gating window 70 are shown. It must be noted that Fig. 3 is not shown in scale, but rather for explanation of the basic principle of the present invention.

For example, at least five gating windows can be provided along the trajectory, preferably twelve, for example, but also any other number of gating windows can be provided.

The term "gating window" indicates that in this temporal window, respective image acquisition is provided, since the gating window refers to a specific, predetermined and thus known phase of the heart cycle.

Within the gating windows 68, 70, the X-ray tube 36 with the two focal spots and the X-ray detector 38 are moved to a number of image acquisition positions, for example six image acquisition positions as shown in Fig. 3, where the respective numbers are used in relation with circles on the trajectory.

In a first position 1, X-ray imaging is provided by the first focal spot 48, and afterwards, an image acquisition is provided from the respective second focal spot 50, which, when the first focal spot 48 is arranged on position 1, is simultaneously arranged at position 1'. Next, the X-ray source, and of course also the X-ray detector 38, is moved along the trajectory to the next position, indicated with 2 and 2'. Here again, a first acquisition is provided by the first focal spot 48 from position 2, and afterwards an image acquisition from the second focal spot 50 from position 2'. This is continued, for example, for points 3/3', 4/4', 5/5', and 6/6' for the respective positions of the first focal spot 48/the second focal spot 50. In other words, instead of having an angular section 72 ranging from position 1 to position 6, an angular range from position 1 to position 6' is provided, due to the additional second focal spot 50 and the respective offset distance 52 along the trajectory. In other words, an additional angular section 76 is provided resulting in an enlarged angular section 74.

From the first gating window 68, the C-arm is moved further on the trajectory 66 to the second gating window 70, where a respective sequence of projection pairs 1/1' etc. is provided, once again providing an enlarged angular section for the gating window.

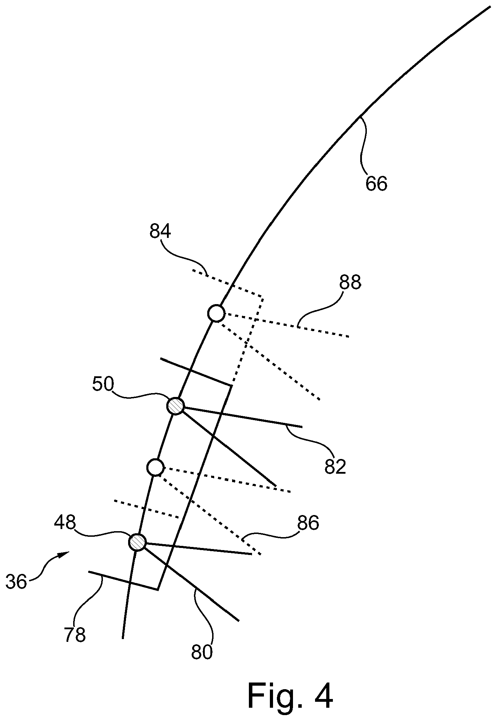

In Fig. 4, the X-ray source 36 is schematically indicated with a half rectangular 78 with the first focal spot 48 resulting in a respective first X-ray beam 80, and the second focal spot 50 resulting in a respective second X-ray beam 82. The first X-ray beam 80 and the second X-ray beam 82 are provided in a sequential manner, and not at the same time. When the X-ray source 36 is moved to the next position within the gating window, wherein the X-ray source is indicated with a dotted line rectangular 84, a further first X-ray beam 86 and a further second X-ray beam 88 are provided, which are also indicated with dotted lines.

Thus, according to the present invention, by providing the second focal spot in the offset direction along the trajectory, image information from a further angular position is provided, thus resulting in an improved and enlarged gating window segment.

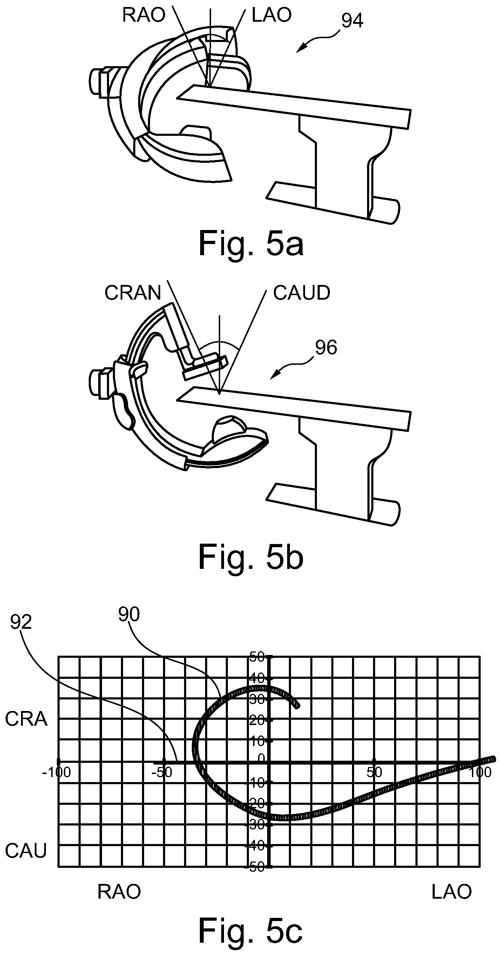

Figs. 5A and 5C show an example for further trajectories, i.e. further rotational movements, in addition to a rotational movement in an even plane. The trajectory according to the invention is a dual axis rotation trajectory, wherein the trajectory is based on a simultaneous propeller and roll movement, as indicated in Figs. 5A and 5B. The resulting curve in space is indicated with a first line 90 in Fig. 5C. Contrary to this, a second line 92 indicates a circular arc as a relatively simple type of rotation. The trajectory 90 may be provided as a so-called "XperSwing trajectory", as used by Philips.

Fig. 5A indicates a propeller movement 94, and Fig. 5B indicates a roll movement 96, which are combined to result in the trajectory 90 shown in Fig. 5C.

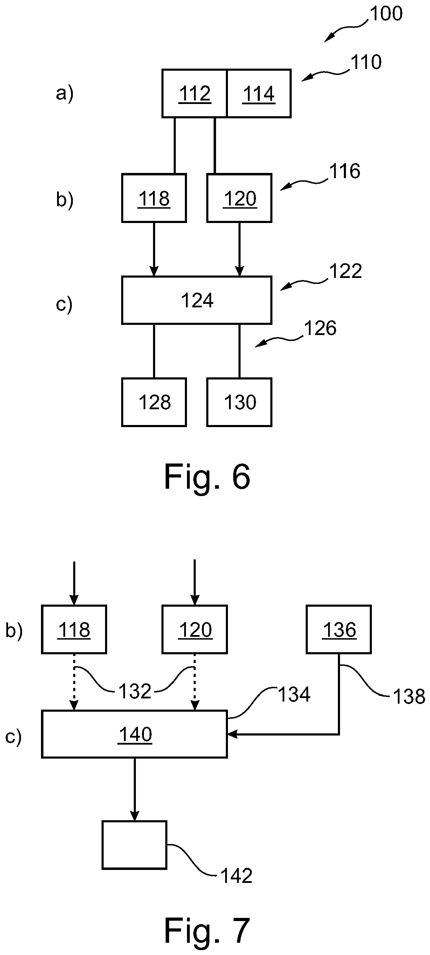

Fig. 6 shows a method 100 for providing tomographic image data of an object, comprising the following steps: In a first step 110, an X-ray source is moved in a moving procedure 112 on a first end of a C-arm structure along a source trajectory. The X-ray source is provided with a first and a second focal spot that are displaced to each other with a focal spot distance in an offset direction, which offset direction is aligned with the trajectory. Simultaneously, in a second moving procedure 114, a detector is moved on a second end of the C-arm structure along a corresponding detector trajectory. In a second step 116, an object is radiated in first X-ray radiation procedure 118 with first X-ray radiation from the first focal spot towards the detector. Alternatively, in a second radiation procedure 120, the object is radiated with second X-ray radiation from the second focal spot towards the detector. In a third step 122, respective X-ray radiation from the first or the second X-ray radiation is detected with the detector in a detecting step 124. Further, respective signals are provided 126 as respective raw image data 128, 130 for the respective focal spot.

The first step 110 is also referred to as step a), the second step 116 as step b), and the third step 122 as step c).

The movement of step a) may be provided continuously. For example, the movement may be provided as a constant and uniform movement while steps b) and c) are performed. For example, in case the duration of the respective X-ray radiation from the respective focal spot positions is very short compared to the movement of the C-arm, the movement during the X-ray image acquisition from one position does not have any further influence on the respective image data.

In other words, steps b) and c) may be provided while step a) is being performed.

As indicated above, in step b), the first and the second X-ray radiation are radiated in an alternating manner.

According to a further example, shown in Fig. 7, the first and second X-ray radiation are provided in a continuous alternating manner 132, which is indicated with dotted arrows leaving the respective boxes. A continuous sampling 134 of raw image data is provided. A gating signal 136 relating to a function of the object is provided, indicated with arrow 138, during the continuous sampling 134 in relation to the raw image data. From the continuously sampled raw image data, raw image data 140 assigned to a predetermined gating signal phase is selected for a reconstruction 142 of three-dimensional image data of the object. This is also referred to as "retrospective" evaluation for sampling.

For example, the gating signal may be an ECG-signal from a patient, and a particular heart phase is predetermined for the assignment to certain raw image data acquired during the respective phase.

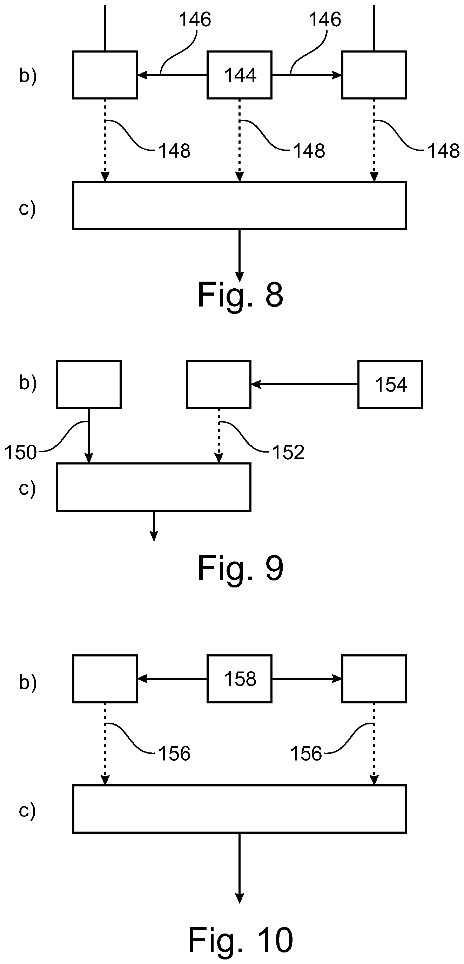

According to a further example, shown in Fig. 8, a gating signal 144 relating to a function of the object is provided, indicated with two arrows 146 during the movement of the C-arm structure and the radiation of the object. The radiation and the detection are arranged only within predetermined gating signal phases, which arrangement is indicated with respective dotted arrows 148. This is also referred to as "prospective" sampling or triggering.

A number of gating windows can be provided along a predefined trajectory length. For example, a trajectory of 180° is provided comprising at least five distributed positions for a gating window, for example twelve gating windows, i.e. twelve positions for a gating window may be provided. In case of a patient, the gating signal may be an ECG signal, as indicated above, and the predetermined gating signal phase is a predetermined section of a heart cycle, for example 20 % of a heart cycle.

According to a further embodiment, shown in Fig. 9, X-ray radiation from one of the two focal spots is provided from a first number of positions across the whole trajectory, which is indicated by a through-line arrow 150. X-ray radiation by the other one of the two focal spots is provided from a second number of positions arranged in a number of gating windows across the whole trajectory, which arrangement only in a number of gating windows is indicated with a dotted arrow 152. A box 154 indicates the provision of the gating signal.

According to a further example, shown in Fig. 10, X-ray radiation from the first focal spot and the second focal spot is provided only from a number of positions arranged in a number of gating windows across the trajectory. This is indicated with two dotted arrows 156, and a further box 158 indicating the gating signal.

According to a further example (not further shown), X-ray radiation from the first focal spot and the second focal spot is provided only from a number of positions arranged in a number of gating windows across the trajectory. For example, the gating windows may be arranged separated from each other.

According to a further example, also not further shown, the moving of step a) is provided in relation to the radiation of step b), such that a pair of a first and a second effective source position is followed by a subsequent pair of a first and second effective source position arranged in a displaced manner, such that one of the effective source positions of the subsequent pair is arranged between the former first and second effective source positions and the other one of the effective source positions of the subsequent pair is arranged outside the displacement of the former first and second effective source positions. For example, this has already been mentioned with respect to Fig. 3.

The first and the second X-ray radiation may be provided as dual energy X-ray radiation. The first X-ray radiation may be provided with a first spectrum, for example, and the second X-ray radiation with a second spectrum, wherein the first and the second spectrum may be provided separated from each other.

According to the present invention, the additional positioning of the second focal spot and the same position of the X-ray tube provides an additional view, or with respect to a sequence of acquisitions in a gating window provides additional views, such that the reconstruction accuracy is increased and improved due to the different geometric information the image, or the images, i.e. the view or views, carry.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A C-arm structure (12) for X-ray imaging, comprising:
- a C-arm (32);
- a movable C-arm support (34);
- an X-ray source (36); and
- an X-ray detector (3 8);
wherein the C-arm comprises a first end (40) and a second end (42), wherein the X-ray source is mounted to the first end and the detector is mounted to the second end; wherein the C-arm is mounted to the C-arm support such that the X-ray source and the detector are movable around an object (44) of interest on respective source and detector trajectories (46) which are dual axis rotation trajectories based on a simultaneous propeller and roll movement of the C-arm;
wherein the X-ray source comprises at least a first focal spot (48) and a second focal spot (50) spaced apart from each other with a focal spot distance (52) in an offset direction (54), which offset direction is aligned with the source trajectory.

2. C-arm structure according to claim 1, wherein the first and the second focal spot are positioned on the trajectory of the X-ray source.

3. C-arm structure according to claim 1 or 2, wherein the offset distance is are adaptable.

4. C-arm structure according to one of the preceding claims, wherein the X-ray source is provided as one stereo X-ray tube with two focal spots.

5. C-arm structure according to one of the preceding claims, wherein the X-ray source is provided as a dual energy X-ray tube.

6. An X-ray imaging system (10), comprising:
- a C-arm structure (12) according to one of the preceding claims;
- a moving arrangement (14) for driving the C-arm structure; and
- a processing unit (16);
wherein the processing unit is configured to control the moving arrangement to actuate the movement of the X-ray source and the detector along the respective trajectories;
wherein the processing unit is configured to control the X-ray source and the generation of X-ray radiation from the first focal spot and the second focal spot; and
wherein the processing unit is configured to control the detector and to receive raw image data from the detector.

7. A method (100) for providing tomographic image data of an object, comprising the following steps:
a) moving (112) an X-ray source on a first end of a C-arm structure along a source trajectory; wherein the X-ray source is provided with a first and a second focal spot which are displaced to each other with a focal spot distance in an offset direction, which offset direction is aligned with the source trajectory; and simultaneously moving (114) a detector on a second end of the C-arm structure along a corresponding detector trajectory; wherein the source and detector trajectories are dual axis rotation trajectories based on a simultaneous propeller and roll movement of the C-arm;
b) radiating (118) an object with first X-ray radiation from the first focal spot towards the detector; or
radiating (120) the object with second X-ray radiation from the second focal spot towards the detector;
c) detecting (124) respective X-ray radiation from the first or the second X-ray radiation with the detector; and providing (126) respective signals as raw image data (128, 130).

8. Method according to claim 7, wherein in step b), the first and the second X-ray radiation are radiated in an alternating manner.

9. Method according to claim 7 or 8, wherein the first and second X-ray radiation are provided in a continuous alternating manner (132) and wherein a continuous sampling (134) of raw image data is provided;
wherein a gating signal (136) relating to a function of the object is provided (138) during the continuous sampling in relation to the raw image data; and
wherein from the continuously sampled raw image data, raw image data (140) assigned to a predetermined gating signal phase is selected for a reconstruction (142) of three-dimensional image data of the object.

10. Method according to claim 7 or 8, wherein a gating signal (144) relating to a function of the object is provided (146) during the movement of the C-arm structure and the radiation of the object; and
wherein the radiation and the detection is arranged (148) only within predetermined gating signal phases.

11. Method according to one of the claims 7 to 10, wherein X-ray radiation from one of the two focal spots is provided (150) from a first number of positions across the whole trajectory; and wherein X-ray radiation by the other one of the two focal spots is provided from a second number of positions arranged (152) in a number of gating windows across the whole trajectory.

12. Method according to one of the claims 7 to 10, wherein X-ray radiation from the first focal spot and the second focal spot is provided only from a number of positions arranged in a number of gating windows across the trajectory.

13. Method according to one of the claims 7 to 12, wherein the offset distance is adapted during the movement of the C-arm.

14. Computer program element for controlling an apparatus according to one of the claims 1 to 6, which, when being executed by a processing unit, is adapted to perform the method steps of one of the claims 7 to 13.

15. Computer readable medium having stored the program element of claim 14.

## Patentansprüche

1. C-Bogen-Struktur (12) zur Röntgenbildgebung, umfassend:
- einen C-Bogen (32);
- eine bewegliche C-Bogen-Halterung (34);
- eine Röntgenquelle (36); und
- einen Röntgendetektor (38);
wobei der C-Bogen ein erstes Ende (40) und ein zweites Ende (42) umfasst, wobei die Röntgenquelle an dem ersten Ende montiert ist und der Detektor an dem zweiten Ende montiert ist; wobei der C-Bogen derartig an der C-Bogen-Halterung montiert ist, dass die Röntgenquelle und der Detektor auf jeweiligen Quellen- und Detektortrajektorien (46) um ein interessierendes Objekt (44) bewegbar ist, bei denen es sich um Doppelachsen-Rotationstrajektorien basierend auf einer gleichzeitigen Anschub- und Wankbewegung des C-Bogens handelt;
wobei die Röntgenquelle mindestens einen ersten Brennfleck (48) und einen zweiten Brennfleck (50) umfasst, die voneinander um einen Brennfleckabstand (52) in einer Versatzrichtung (54) beabstandet sind, wobei die Versatzrichtung auf die Quellentrajektorie ausgerichtet ist.

2. C-Bogen-Struktur nach Anspruch 1, wobei der erste und der zweite Brennfleck auf der Trajektorie der Röntgenquelle positioniert sind.

3. C-Bogen-Struktur nach Anspruch 1 oder 2, wobei der Versatzabstand anpassbar ist.

4. C-Bogen-Struktur nach einem der vorhergehenden Ansprüche, wobei die Röntgenquelle als eine Stereo-Röntgenröhre mit zwei Brennflecken vorgesehen ist.

5. C-Bogen-Struktur nach einem der vorhergehenden Ansprüche, wobei die Röntgenquelle als eine Dualenergie-Röntgenröhre vorgesehen ist.

6. Röntgenbildgebungssystem (10), umfassend:
- eine C-Bogen-Struktur (12) nach einem der vorhergehenden Ansprüche;
- eine Bewegungsanordnung (14) zum Antreiben der C-Bogen-Struktur; und
- eine Verarbeitungseinheit (16);
wobei die Verarbeitungseinheit zum Steuern der Bewegungsanordnung konfiguriert ist, um die Bewegung der Röntgenquelle und des Detektors entlang der jeweiligen Trajektorien zu bewirken;
wobei die Verarbeitungseinheit konfiguriert ist, um die Röntgenquelle und die Erzeugung von Röntgenstrahlung von dem ersten Brennfleck und dem zweiten Brennfleck aus zu steuern; und
wobei die Verarbeitungseinheit konfiguriert ist, um den Detektor zu steuern und Rohbilddaten von dem Detektor zu empfangen.

7. Verfahren (100) zum Bereitstellen von tomographischen Bilddaten eines Objekts, wobei das Verfahren die folgenden Schritte umfasst:
a) Bewegen (112) einer Röntgenquelle an einem ersten Ende einer C-Bogen-Struktur entlang einer Quellentrajektorie; wobei die Röntgenquelle mit einem ersten und einem zweiten Brennfleck vorgesehen ist, die zueinander um einen Brennfleckabstand in einer Versatzrichtung versetzt sind, wobei die Versatzrichtung auf die Quellentrajektorie ausgerichtet ist; und gleichzeitiges Bewegen (114) eines Detektors an einem zweiten Ende der C-Bogen-Struktur entlang einer entsprechenden Detektortrajektorie; wobei die Quellen- und die Detektortrajektorie Doppelachsen-Rotationstrajektorien basierend auf einer gleichzeitigen Anschub- und Wankbewegung des C-Bogens sind;
b) Bestrahlen (118) eines Objekts mit einer ersten Röntgenstrahlung von dem ersten Brennfleck aus in Richtung auf den Detektor zu; oder
Bestrahlen (120) eines Objekts mit einer zweiten Röntgenstrahlung von dem zweiten Brennfleck aus in Richtung auf den Detektor zu;
c) Detektieren (124) der jeweiligen Röntgenstrahlung von der ersten oder der zweiten Röntgenstrahlung mit dem Detektor; und Bereitstellen (126) jeweiliger Signale als Rohbilddaten (128, 130).

8. Verfahren nach Anspruch 7, wobei in Schritt b) die erste und die zweite Röntgenstrahlung auf abwechselnde Weise abgestrahlt werden.

9. Verfahren nach Anspruch 7 oder 8, wobei die erste und die zweite Röntgenstrahlung auf eine kontinuierliche Weise (132) bereitgestellt werden und wobei eine kontinuierliche Abtastung (134) von Rohbilddaten bereitgestellt wird;
wobei ein Gating-Signal (136) bezüglich einer Funktion des Objekts während der kontinuierlichen Abtastung in Bezug auf die Rohbilddaten bereitgestellt wird (138); und
wobei anhand der kontinuierlich abgetasteten Rohbilddaten Rohbilddaten (140), die einer vorgegebenen Gating-Signal-Phase zugewiesen sind, zur Rekonstruktion (142) von dreidimensionalen Bilddaten des Objekts ausgewählt werden.

10. Verfahren nach Anspruch 7 oder 8, wobei ein Gating-Signal (144) bezüglich einer Funktion des Objekts während der Bewegung der C-Bogen-Struktur und der Bestrahlung des Objekts bereitgestellt wird (146); und
wobei die Bestrahlung und die Detektion nur innerhalb vorgegebener Gating-Signal-Phasen vorgesehen sind (148).

11. Verfahren nach einem der Ansprüche 7 bis 10, wobei Röntgenstrahlung von einem der beiden Brennflecke von einer ersten Anzahl von Positionen über die gesamte Trajektorie hinweg bereitgestellt wird (150); und wobei Röntgenstrahlung durch den anderen der beiden Brennflecke von einer zweiten Anzahl von Positionen bereitgestellt wird, die in einer Anzahl von Gating-Fenstern über die gesamte Trajektorie hinweg angeordnet sind (152).

12. Verfahren nach einem der Ansprüche 7 bis 10, wobei Röntgenstrahlung von dem ersten Brennfleck und dem zweiten Brennfleck nur von einer Anzahl von Positionen bereitgestellt wird, die in einer Anzahl von Gating-Fenstern über die Trajektorie hinweg angeordnet sind.

13. Verfahren nach einem der Ansprüche 7 bis 12, wobei der Versatzabstand während der Bewegung des C-Bogens angepasst wird.

14. Computerprogrammelement zum Steuern eines Geräts nach einem der Ansprüche 1 bis 6, das, wenn es durch eine Verarbeitungseinheit ausgeführt wird, dafür ausgelegt ist, die Verfahrensschritte nach einem der Ansprüche 7 bis 13 durchzuführen.

15. Computerlesbares Medium, auf dem das Programmelement aus Anspruch 14 gespeichert ist.

## Revendications

1. Structure à bras en C (12) pour imagerie à rayons X, comprenant :
- un bras en C (32) ;
- un support de bras en C mobile (34) ;
- une source de rayons X (36) ; et
- un détecteur de rayons X (38) ;
dans laquelle le bras en C comprend une première extrémité (40) et une seconde extrémité (42), dans laquelle la source de rayons X est montée sur la première extrémité et le détecteur est monté sur la seconde extrémité ; dans laquelle le bras en C est monté sur le support de bras en C de telle sorte que la source de rayons X et le détecteur soient mobiles autour d'un objet (44) d'intérêt sur des trajectoires respectives de source et de détecteur (46) qui sont des trajectoires de rotation biaxiales en fonction d'un mouvement simultané de propulsion et de roulis du bras en C ;
dans laquelle la source de rayons X comprend au moins un premier foyer (48) et un second foyer (50) espacés l'un de l'autre avec une distance de foyers (52) dans une direction de décalage (54), laquelle direction de décalage est alignée avec la trajectoire de source.

2. Structure à bras en C selon la revendication 1, dans laquelle les premier et second foyers sont positionnés sur la trajectoire de la source de rayons X.

3. Structure à bras en C selon la revendication 1 ou 2, dans laquelle la distance de décalage est adaptable.

4. Structure à bras en C selon l'une des revendications précédentes, dans laquelle la source de rayons X est prévue sous forme de tube singulier à rayons X stéréo avec deux foyers.

5. Structure à bras en C selon l'une des revendications précédentes, dans laquelle la source de rayons X est prévue sous forme de tube à rayons X à double énergie.

6. Système d'imagerie à rayons X (10), comprenant :
- une structure à bras en C (12) selon l'une des revendications précédentes ;
- un agencement mobile (14) pour entraîner la structure à bras en C ; et
- une unité de traitement (16) ;
dans lequel l'unité de traitement est configurée pour commander l'agencement mobile pour actionner le mouvement de la source de rayons X et du détecteur le long des trajectoires respectives ;
dans lequel l'unité de traitement est configurée pour commander la source de rayons X et la génération de rayonnement de rayons X à partir du premier foyer et du second foyer ; et
dans lequel l'unité de traitement est configurée pour commander le détecteur et pour recevoir des données d'image brutes à partir du détecteur.

7. Procédé (100) pour fournir des données d'image tomographiques d'un objet, comprenant les étapes suivantes :
a) le mouvement (112) d'une source de rayons X sur une première extrémité d'une structure à bras en C le long d'une trajectoire de source ; dans lequel la source de rayons X est pourvue de premier et second foyers qui sont déplacés l'un par rapport à l'autre avec une distance de foyers dans une direction de décalage, laquelle direction de décalage est alignée avec la trajectoire de source ; et le mouvement simultané (114) d'un détecteur sur une seconde extrémité de la structure à bras en C le long d'une trajectoire de détecteur correspondante ; dans lequel les trajectoires de source et de détecteur sont des trajectoires de rotation biaxiales en fonction d'un mouvement simultané de propulsion et de roulis du bras en C ;
b) l'irradiation (118) d'un objet avec un premier rayonnement de rayons X à partir du premier foyer vers le détecteur ; ou
l'irradiation (120) de l'objet avec un second rayonnement de rayons X à partir du second foyer vers le détecteur ;
c) la détection (124) de rayonnement de rayons X respectif à partir du premier ou du second rayonnement de rayons X avec le détecteur ; et la fourniture (126) de signaux respectifs sous forme de données d'image brutes (128, 130).

8. Procédé selon la revendication 7, dans lequel, dans l'étape b), les premier et second rayonnements de rayons X sont irradiés de manière alternée.

9. Procédé selon la revendication 7 ou 8, dans lequel les premier et second rayonnements de rayons X sont fournis de manière alternée continue (132) et dans lequel un échantillonnage continu (134) de données d'image brutes est prévu ;
dans lequel un signal de déclenchement (136) concernant une fonction de l'objet est fourni (138) durant l'échantillonnage continu par rapport aux données d'image brutes ; et
dans lequel, à partir des données d'image brutes échantillonnées en continu, des données d'image brutes (140) attribuées à une phase prédéterminée de signal de déclenchement sont sélectionnées pour une reconstruction (142) de données d'image tridimensionnelles de l'objet.

10. Procédé selon la revendication 7 ou 8, dans lequel un signal de déclenchement (144) concernant une fonction de l'objet est fourni (146) durant le mouvement de la structure à bras en C et l'irradiation de l'objet ; et
dans lequel l'irradiation et la détection sont agencées (148) seulement au sein de phases prédéterminées de signal de déclenchement.

11. Procédé selon l'une des revendications 7 à 10, dans lequel le rayonnement de rayons X à partir de l'un des deux foyers est fourni (150) à partir d'un premier nombre de positions sur la trajectoire entière ; et dans lequel le rayonnement de rayons X par l'autre des deux foyers est fourni à partir d'un second nombre de positions agencées (152) dans un nombre de fenêtres de déclenchement sur la trajectoire entière.

12. Procédé selon l'une des revendications 7 à 10, dans lequel le rayonnement de rayons X à partir du premier foyer et du second foyer est fourni seulement à partir d'un nombre de positions agencées dans un nombre de fenêtres de déclenchement sur la trajectoire.

13. Procédé selon l'unes des revendications 7 à 12, dans lequel la distance de décalage est adaptée durant le mouvement du bras en C.

14. Élément de programme d'ordinateur pour commander un appareil selon l'une des revendications 1 à 6, qui, lorsqu'il est exécuté par une unité de traitement, est adapté pour effectuer les étapes de procédé selon l'une des revendications 7 à 13.

15. Support lisible par ordinateur possédant, stocké, l'élément de programme selon la revendication 14.
